# EUROPEAN PATENT APPLICATION

(11) **EP 1 671 929 A2**
(43) Date of publication of application: **21.06.2006**
(21) Application number: 05027208.7
(22) Date of filing: 13.12.2005
(51) Int. Cl.: C01B 33/14

(54) **Process for production of aqueous colloid of graphite silica particles emitting far-infrared rays**

(30) Priority: 17.12.2004 JP 2004365651
(71) Applicant: Faith Niiichi, K.K., Hamamatsu-shi Shizuoka (JP)
(72) Inventor: Suzuki, Nichinobu, Hamamatsu-shi Shizuoka-ken (JP)
(74) Representative: Kirschner, Klaus Dieter

(57) **Abstract**

To prevent or treat diseases in which active oxygen is involved such as inflammatory cell injury, diabetic diseases (nephropathy, retinopathy, and neuropathy), ischemia and reperfusion injury, renal failure, rheumatoid arthritis, and myocardial infarction, a process for producing an aqueous colloid of graphite silica particles emitting far-infrared rays, the process comprising adding graphite silica (black silica) particles emitting far-infrared rays to water, is provided.

## Description

### BACKGROUND OF THE INVENTION

### Field of the invention

The present invention relates to a process for producing an aqueous colloid containing graphite silica particles used for activating the body, and particularly to a process employing graphite silica particles emitting far-infrared rays.

The present invention has wide applications and those alone now found include the fields described below. It exerts excellent effect not only in such fields but also in any field associated with a human life, and also includes a sufficient possibility of being used in new areas in the future.

The application of the aqueous colloid of graphite silica particles prepared according to the present invention covers, for example, the field of medicine for brain, nerve, cerebrovascular, lung, gut mucosa, liver, kidney, eye, skin, diabetic, and immune diseases or disorders and particularly for cancers such as large intestine, small intestine, and skin cancers, and the field of improvement of adult diseases and the like such as diabetes, cytopathy, skin aging, and increased lipid peroxide or neutral fat.

There are also included the field of prevention of hypertension, the field of improvement of myocardial infarction, angina pectoris, cerebral infarction, intracerebral hemorrhage, ulcer, and, further, life-style related diseases, i.e. lately talked about pollen disease, atopy, and stress, and the field of suppression of harmful effect of electromagnetic wave.

Particularly, according to the present invention, the aqueous colloid of graphite silica particles emitting far-infrared rays can be used as antioxidant by mixing e.g. in water to drink as medicine, and are also suitable for the field of a pH regulator and powdered and softening agents.

In addition, the aqueous colloid can be used by mixing in so-called drinkable preparations and soft drinks as healthy drinks, or as an additive for preparations containing a flavor or a coloring agent for various human, livestock, and pet foods, or in the fields of various health foods, production of various cosmetics, bath agents, and the like.

Description of the Related Art

The graphite silica (black silica) is found only upstream of the Amano river located on Kaminokuni-cho, Hiyama-gun, Hokkaido, Japan. The graphite silica is a natural ore assumed to have been generated by the way that diatoms on the sea-bottom piled up over a period of several hundreds million years and heaved on the earth's surface, and is known to contain various natural minerals abundantly and make the radiant divergence of various active waves including far-infrared rays.

The graphite silica absorbs solar energy by several times the power of substances generated on the earth's surface and semipermanently repeats the radiation.

Among other far-infrared rays, the above far-infrared rays are stronger in waves of 4 microns to 14 microns which activate living cells at ordinary temperature. Far-infrared rays having these waves are called growth rays, and make a resonance action on cellular molecules of a living organism to activate the blood and the cell in themselves, thereby facilitating the systemic metabolism and enhancing life. The graphite silica is a natural ore (designated as a legal ore by the Mining Division, the Hokkaido Bureau of Industrial Trade and Industry) emitting a high percentage of far-infrared ray to achieve a powerful sterilization effect and an odor eliminating effect through unionization.

The above conventional technology applied as antioxidant permits the presence of a radical generated as a metabolic intermediate in an oxidation-reduction system or a radical produced by ultraviolet light, radiation, city water chlorine, some agricultural chemicals, some medicines, photocatalytic activity positive cosmetics, tobacco, or the like. As typical radicals, there is known the presence of active oxygens such as superoxide anion (O²⁻) and hydroxyl radical (OH*).

Active oxygens play an essential role in a biophylactic mechanism against viruses or pathogenic bacteria; particularly, superoxide anion and the like in hormone biosynthesis or gene expression. At the same time, however, active oxygens such as superoxide anion and hydroxy radical which are radicals per se and those such as hydrogen peroxide and singlet oxygen which become a source for radicals are known to induce, when generated too much, cell membrane injury, protein denature, gene mutation, and the like by reacting with organism components such as lipid, protein, and nucleic acid, resulting in damaging vital functions and producing various diseases such as cancer or senescence.

Diseases in which such active oxygens are involved include, for example, inflammatory cell injury, diabetic diseases (nephropathy, retinopathy, and neuropathy), ischemia and reperfusion injury, renal failure, rheumatoid arthritis, and myocardial infarction.

Substances which have the properties of trapping and stabilizing radicals in a living body are called radical scavengers. In the living body, enzymes such as superoxide dismutase (SOD), catalase, and glutathione peroxidase (GSH-PX) as substances controlling the generation of active oxygens, and compounds such as vitamin C, vitamin E, carotenoid, uric acid, and bilirubin as substances having the properties of trapping active oxygens to inhibit chain reaction are known to act as radical scavengers . In addition to in vivo substances, reagents such as isoflavonoid, phenols, and ascorbic acid are known to have an eliminating action on active oxygens.

Substances which have the properties of trapping and stabilizing radicals in a living body are called radical scavengers. In the living body, enzymes such as superoxide dismutase (SOD), catalase, and glutathione peroxidase (GSH-PX) as substances controlling the generation of active oxygens and compounds such as vitamin C, vitamin E, carotenoid, uric acid, and bilirubin as substances having the properties of trapping active oxygens to inhibit chain reaction are known to act as radical scavengers.

In addition to in vivo substances, reagents such as isoflavonoid, phenols, and ascorbic acid are known to have an eliminating action on active oxygens. Active oxygen species generated too much in a living body generally have high chemical reactivity, easily react with neighboring components such as lipid, nucleic acid, and protein in the living body, and produce oxidative disturbances resulting in various diseases. Their oxidation by superoxide, one kind of active oxygen species, leads to the formation of foam cells, causing arterial sclerosis.

The generation of hydroxy radical resulting from irradiation causes serious disturbance such as cancer initiation for a living body.

As the toxicity of such active oxygen species against a living body becomes apparent, antioxidants including active oxygen species-eliminating substances having the activity efficiently removing the species is expected to serve as preventive agents against the oxidative deterioration of components contained in the living body or in foods, medicines, agricultural products, and the like, and to be practically used in the fields of food industry, particularly processed marine products, health foods, as well as nutritional foods, medicine and agriculture, and cosmetics. (Halliwell B. and Gutterridge M.C. BIOCHEM.J. 219, 1-1 4, (1984))

By way of example, a conventional skin care preparation containing that extract derived from an olive plant (excluding leaves) which is contained as a skin-beautifying ingredient, especially as a skin aging-preventing ingredient and/or a bleaching ingredient has been previously invented (Japanese Patent Laid-Open No. 2001-181198 (Abstract)). The extract can be obtained by subjecting, to extraction treatment with water and/or an organic solvent, the olive plant and/or a product obtained in a process for producing olive oil, and the effects can be enhanced by concentrating the extract and/or fractionating/purifying the extract. Skin-beautifying agents, especially a skin aging-preventing agent and a bleaching agent, containing the extract as active ingredient have been also provided.

Similarly, a hydrous cosmetic effective for mitigating dermal inflammatory reaction due to peroxidation reaction in the skin and improving dermal conditions (wrinkles or tension of the skin) has been invented (Japanese Patent Laid-Open No. 05-009108 (Abstract)). The cosmetic contains, as essential components, 0.00001 to 0.1 wt%, on a selenium basis, of a selenium compound and/or plant extract containing the compound and 40 wt% or more of water, in the form e. g. of a solubilization type, oil-in-water emulsion, water-in-oil emulsion, viscous or gelled preparation.

In addition, a medicine composition for treating or inhibiting disease conditions induced by free radicals has been invented (Japanese Patent Laid-Open No. 09-169650 (Abstract)). The composition comprisesan antioxidizing dose of 8,9-dehydroestrone or its pharmaceutically acceptable 3-ester sulfate salt.

The present inventors have previously invented a nano-ceramic particle composed of a ceramic particle comprising silicon carbide and/or its unreacted matter, which can exert excellent effect in the medicinal field of antioxidant or in preventing or treating any disease, and has filed a patent application about the particle (Japanese Patent Application No. 2004-042137). The present invention has been achieved by further improving this invention.

### SUMMARY OF THE INVENTION

Antioxidants which now appear on a market or have been previously invented have, as yet, insufficient effect in treating or preventing diseases in which active oxygens are involved such as inflammatory cell injury, diabetic diseases (nephropathy, retinopathy, and neuropathy), ischemia and reperfusion injury, renal failure, rheumatoid arthritis, and myocardial infarction. Particularly, the appearance of any substance having significant effect all over the above described fields has not yet been proposed.

The present invention features a process for producing an aqueous colloid of graphite silica particles emitting far-infrared rays, the process comprising adding graphite silica (black silica) particles to water.

Another feature of the present invention is that the above described graphite silica (black silica) particles are composed of 4 to 6% of carbon, 0.35 to 0.55% of magnesium, 70 to 90% of silica, 1.0 to 2.0% of titanium, 0.4 to 0.6% of iron dioxide, 0.06 to 0.1% of sodium, 5 to 8% of aluminum, 0.8 to 2.0% of potassium, 0.01 to 0.03% of calcium, and 0.25 to 0.4% of water.

Another feature of the present invention is that the above described graphite silica (black silica) particles emit far-infrared rays with an emissivity of 90 to 99% at ordinary temperature, and emit so-called growth rays or health rays in a wavelength range of 2 microns to 18 microns.

Another feature of the present invention is that the above described graphite silica emitting far-infrared rays has a size of 0.3 micron or less.

Another feature of the present invention is that the above described graphite silica emitting far-infrared rays has a size of 0.3 micron or less.

Another feature of the present invention is to provide a process for producing an aqueous colloid, wherein the graphite silica emitting far-infrared rays has a size of 0.3 micron or less, and the aqueous colloid is prepared by dissolving the graphite silica in, for example, normal water, and filtering the solution.

Another feature of the present invention is that the graphite silica emitting far-infrared rays used in the above described aqueous colloid containing graphite silica particles emitting far-infrared rays is prepared by forming the material into a spherical shape.

Another feature of the present invention is that the above described aqueous colloid containing graphite silica particles emitting far-infrared rays is used as an antioxidant .

Another feature of the present invention is that the above described aqueous colloid containing graphite silica particles emitting far-infrared rays is used in anti-inflammatory agents.

Another feature of the present invention is that the above described aqueous colloid containing graphite silica particles emitting far-infrared rays is used in analgesics.

Another feature of the present invention is that the above described aqueous colloid of graphite silica particles emitting far-infrared rays is used in anti-cancer agents.

Another feature of the present invention is that the above described aqueous colloid containing graphite silica particles emitting far-infrared rays is used in inhibitors for aldose reductase associated with diabetic complications.

Another feature of the present invention is that the above described aqueous colloid containing graphite silica particles emitting far-infrared rays is used in disaccharidase inhibitors.

Another feature of the present invention is that the above described aqueous colloid containing graphite silica particles emitting far-infrared rays is used in melanin production inhibitors.

Another feature of the present invention is that the above described aqueous colloid containing graphite silica particles emitting far-infrared rays is used in anti-allergic agents.

Another feature of the present invention is that the above described aqueous colloid of graphite silica particles emitting far-infrared rays is used in a mixture with healthy drinks such as drinkable preparations or soft drinks, for example.

An advantage of the present invention is that the graphite silica particles can be mixed abundantly in water to provide e. g. humans with the mixture in the form of the so-called mineral water. The mineral water is readily absorbable in the human body and, when drunk, can facilitate the systemic metabolism; the nutrient thereof ismicronized into smaller sizes, ionized, and absorbed to allow immunizing power inherent in human beings to be recovered, has an excellent effect of warming the body, can lead to a good sleep, and is good for all disease.

In addition, the above described graphite silica particle emitting the far-infrared rays among other far-infrared rays is the one and only matter available in the world that emits, at ordinary temperature, rays strong in wave lengths of 4 microns to 14 microns activating a living cell; far-infrared rays having such wave lengths are called growth rays, and make resonance action on cellular molecules in a living organism to activate the blood and cell themselves.

Further, mixing the above described graphite silica particles emitting the far-infrared rays in water to use the mixture in humans by the way e.g. of drinking can provide not only, particularly a refreshing drink which is palatable, brisk, and quaffable, but also so-called "very tasty water" as a thinning water for alcoholic beveragesor concentratedjuices.

The aqueous colloid of graphite silica particles prepared according to the present invention is, for example in the field of medicine, capable of obviating the occurrence of various cancers such as large intestine, small intestine, and skin cancers and excellent in effects on brain, nerve, cerebrovascular, lung, gut mucosa, liver, kidney, eye, skin, diabetic, and immune diseases or disorders, and, in the field of adult diseases and the like, can prevent diabetes, cytopathy, skin aging, and increased lipid peroxide or neutral fat.

The aqueous colloid can make the vessel clean, inhibit an increase in cholesterol, and prevent hypertension, which may lead to the prevention of myocardial infarction, angina pectoris, cerebral infarction, intracerebral bleeding, and ulcer. In addition, it can suppress lately talked about life-style related diseases such as pollen disease, atopy, and stress, and harmful effects of electromagnetic waves, and can prevent sleeplessness and oversensitiveness to cold. Particularly, according to the present invention, the aqueous colloid of graphite silica particles emitting far-infrared rays can be used as antioxidant by mixing e.g. in water to drink as medicine, or also employed suitably in a pH regulator and powdered and softening agents.

The aqueous colloid can also be suitably used by mixing in the so-called drinkable preparation and refreshing drink as healthy drinks, or employed in the form of an additive for preparations containing a flavor or a coloring agent for various human, livestock, or pet foods, or in various health foods.

In addition, according to the present invention, the graphite silica particles emitting the far-infrared rays and the aqueous colloid of graphite silica particles can be optimally mixed in various cosmetics and bath agents, and have been reported to thereby whiten the skin (beautiful skin) .

The aqueous colloid of graphite silica particles emitting the far-infrared rays can be easily prepared simply by dissolving the graphite silica in ordinary water or the like followed by filtration, and, by forming the raw material into a spherical shape, becomes highly permeable, convenient, and effective in using for the prevention or treatment of all disease.

For example, in current traditional medical care, the graphite silica some 1 micron in size has been found to have a beneficial effect on burns and to have the effect e.g. of suppressing diarrhea.

The aqueous colloid of graphite silica particles emitting the far-infrared rays of the present invention has been used in analgesics, anti-inflammatory agents, anti-cancers, inhibitors of aldose reductase associated with diabetic complications, disaccharidase inhibitors, melanin production inhibitors, and anti-allergic agents to achieve excellent effects.

Further, for example, it has been confirmed that the aqueous colloid of graphite silica particles has the effect of reducing blood glucose level. Since its antioxidant action is a physical action and the mechanism has reduced the weight of lipid peroxide, it can be said that the trapping of O in hydrogen peroxide leads to the antioxidant action. For example, since it can effectively prevent tissue cell injury due to free radicals, the aqueous colloid can effect e.g. the prevention of skin aging and the suppression of skin cancer.

On the other hand, anti-inflammatory, analgesic, and anti-cancer actions and the like are based on physiological functions, and the mechanism developing such actions are therefore diverse. From many past experimental results, it has been found that the mechanism, together with the action of the aqueous colloid of graphite silica particles, is highly useful as an anti-cancer agent, or as a therapeutic, preventive, or emollient for treatment use.

Further, the results of many clinical tests show that the antioxidant action obtained according to the present invention has cured cold constitution, has removed readiness to fatigue, and has alleviated breath shortness. From these experimental data, medical speculation is readily made because, for example, the antioxidant action is easily associated with the removing of readiness to fatigue.

Typical diseases for which the present invention is intended include various cancers, particularly large intestine, small intestine and skin cancers which have been demonstrated, in many tests, to be prevented by the present invention.

In addition, it has been demonstrated that excellent effects are also achieved against various brain diseases, particularly nervous diseases (Alzheimer disease, Parkinson disease, cerebrovascular disease, etc.), lung diseases (pulmonary oxygen intoxication, respiratory distress syndrome, airway disease, etc.), gut mucosal damages (digestive ulcer, etc.), liver disease, renal diseases (nephritis, renal insufficiency, etc.), eye diseases (cataract, retinal disease, etc.),skin diseases (dermatitis, photosensitivity, etc.), diabetic disease, and immune diseases (AIDS, autoimmune disease, etc.).

Further, in adult disease such as diabetes, the aqueous colloid has excellent effects against glucosidase and aldose reductase and enhances insulin effect. Since it has the effect of preventing cell injury (due to free radicals) and thereby preventing skin aging, the occurrence of blotch or macula can be prevented e.g. in the aged. The aqueous colloid prevents increases in lipid peroxide and neutral fat induced by active oxygen, leading to physical condition improvement such as removed readiness to fatigue.

The aqueous colloid inhibits an increase in unsaturated fatty acid to suppress an increase in cholesterol to make the vessel clean to prevent hypertension, leading to the prevention of myocardial infarction, angina pectoris, cerebral infarction, and intracerebral bleeding, and prevents ulcer.

Further advantage of the present invention is to inhibit symptoms of lately talked about life-style related diseases such as pollenosis and atopy and to prevent obesity, constipation, rheumatism, virus disease, and dropsical swelling. Also, the aqueous colloid has a general detoxication effect, and prevents various allergy. In addition, it has been reported that the aqueous colloid has the effects of relieving stress, mitigating grief, nerve strain, and rage, and suppressing pantophobia and symptoms due to the adverse effects of a personal computer and the like on the body.

The aqueous colloid also prevents the so-called dotage, has a significant microbicidal effect to inhibit diarrhea, cures sleeplessness, and prevents oversensitiveness to cold. Further, it has been reported, for example, that when the aqueous colloid of graphite silica particles was drunk, cold got over the next day after catching, during which only sneezes and coughs occurred without fever, athlete's foot or breath shortness cured, the eye was relieved from fatigue and became clear, and gray hair turned blackish.

### BRIEF DESCRIPTION OF THE DRAWINGS

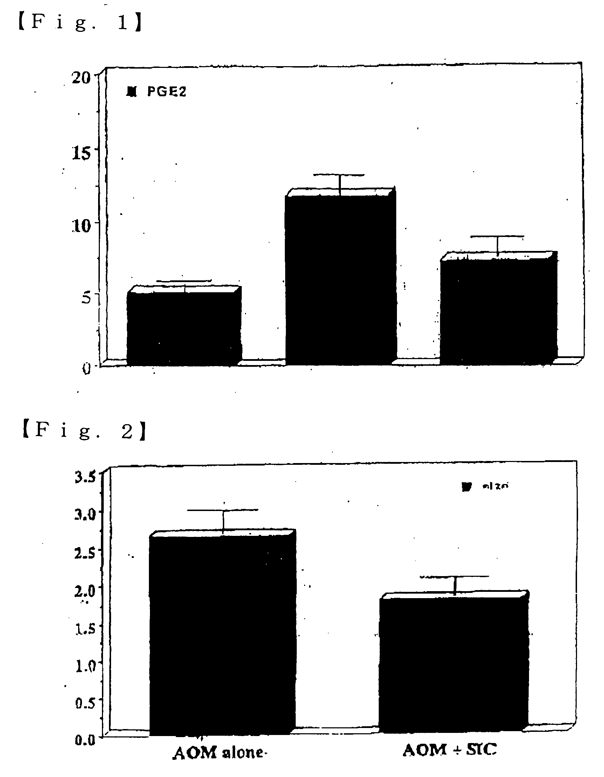

FIG. 1 is a graph showing the effect of suppressing tumor cell proliferation;
FIG. 2 is a graph as to the number of ACF indicating precancerous lesions;

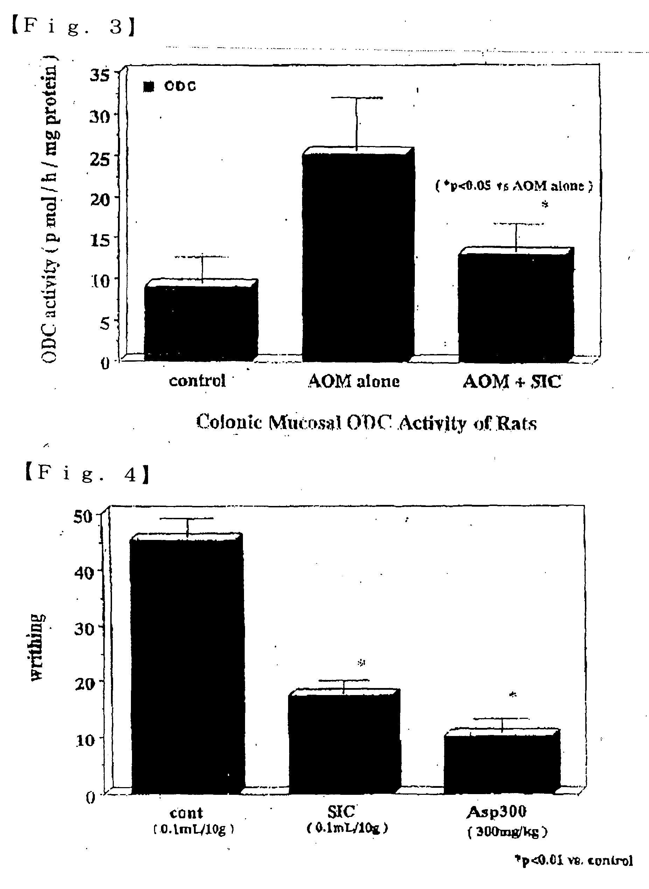

FIG. 3 is a graph showing ODC activity values in a precancerous lesion;
FIG. 4 is a graph in which the frequency of writhing was started to count 10 minutes after administration of acetic acid to mice and recorded during a period of 10 minutes;
FIG. 5 is a graph showing the effect of suppressing carrageenan-induced foot edema in rats;
FIG. 6 is a graph showing an analgesic effect against brewer's yeast-induced inflammatory pain;
FIG. 7 is a graph showing an anti-inflammatory effect;
FIG. 8 is a graph showing an anti-diabetic effect in spontaneous diabetic rats;
FIG. 9 is a graph showing effects of maltose ingestion on insulin secretion;
FIG. 10 is a graph showing effects of maltose ingestion on insulin secretion; and
FIG. 11 is a graph showing an effect against offending bacteria of caries.

### DETAILED DESCRIPTION OF THE PREFERRED EMBODIMENTS

The graphite silica used in the present invention is naturally produced, and consists of 5% of carbon, 80% of silica (SiO₂) , 5% of aluminum, 2% of iron, and 2% of other components such as calcium and magnesium, and, as physical and chemical characteristics, has mineral properties, mild acidity (pH5.5 to 7.5), incombustibility, water resistance, abrasive resistance (hardness 7. 0 to 7.5), high absorption of infrared ray, fragility, and electrical conductivity.

More specifically, the graphite silica consists most preferably of 5.03% of carbon, 0.45% of magnesium, 81.35% of silica, 1.18% of titanium, 0.53% of iron dioxide, 0.08% of sodium, 6.35% of aluminum, 1.66% of potassium, 0.02% of calcium, and 0.31% of water.

The size of the graphite silica is 0.3 micron or less, and particularly a size in the order of nanometers or a size close to it. Using the high frequency electromagnetic wave absorption characteristics of the graphite silica, studies are performed on the synthesis of a graphite silica particle emitting more excellent far-infrared rays by irradiating with a high frequency electromagnetic wave having a frequency of 2 GHz or higher employing a high frequency electromagnetic wave irradiation device.

The production device or means enables the synthetic reaction to be carried out in an extremely short period of time using the raw material itself as a heat generator.

This device encompasses the raw material with a highly fire-resisting, highly heat-insulating material to minimize the scattering, to the environment, of heat energy necessary for the synthetic reaction, and to irradiate the material with the high frequency electromagnetic wave having a frequency of 2 GHz or higher to allow the heat energy to be supplied from the inside of the rawmaterial until the synthetic reaction is completed. In this study, the graphite silica particle emitting excellent far-infrared rays can be therefore prepared in a relatively short period of time.

In the reaction process, irradiating the raw material of graphite silica with the high frequency electromagnetic wave having a frequency of 2 GHz or higher is thought to allow the electromagnetic wave energy to be absorbed to change the energy into an additional heat energy.

Then, an appropriate amount of the graphite silica particle emitting far-infrared rays according the present invention is mixed in easy-to-obtain water including ordinary city water, and the mixture is well stirred to make an aqueous colloid.

Here, various healthy drinks or liquids such as orange juice may be used in place of the "water" described above.

For the graphite silica particles emitting the far-infrared rays and the aqueous colloid of graphite silica particles of the present invention, it has been shown how excellent the antioxidant activity against hydrogen peroxide is, for example in an anti-oxidation test on the aqueous colloid containing graphite silica particles using a rat liver homogenate (this will be described in detail in the section of Tests and Results). In the test, 0.1 ml of a reference liquid of the aqueous colloid of graphite silica particles was added to 0.5 ml of a rat liver homogenate prepared by the law of the art, followed by incubation at 37°C for 10 minutes.

Then, 0.2 ml of hydrogen peroxide (final concentration: 6 mM) and 0.2 ml of iron sulfate (final concentration: 6 mM) were added, and the mixture was stirred, followed by incubation at 37°C for 20 minutes. In a control group, when ultrapure water and vitamin E (25 µg/ml) were used in equal amounts, about 3 to 10 µ of whisker (needle single crystal) was observed. Its feature is that, while common silicon carbide exhibits no magnetism in a semiconductor, it had electrical conductivity on the order of KΩ and showed magnetism.

In Examples of the present invention, the liquid obtained by, as described above, dissolving, in ordinary water, the graphite silica particles emitting the far-infrared rays followed by filtration is referred to as an aqueous colloid of graphite silica particles. The aqueous colloid of graphite silica particles has surface-active activity and amphipathicity (hydrophilicity and lipophilicity), and the hydrogen ion concentration thereof showed a weak acidity of about pH 6.0.

### Test 1

An anti-fatigue effect of the aqueous solution containing the graphite silica (black silica) was first examined.

Silicon has the effects of encouraging the prevention of arteriosclerosis, the organization of the bone, and, further, the activation of a cell. The graphite silica has the effects of activating the blood and cell themselves, accelerating systemic metabolism, and increasing vital force, and thus is thought to have an indirect anti-fatigue effect. Therefore, a test to determine forced swimming time was carried out using the aqueous solution containing the graphite silica.

A test solution was prepared by adding 10% of graphite silica powder based on the amount of purified water, and, 1, 3, and 5 days after the addition, the supernatant was used for the test.

In the determination of forced swimming time in weight-loaded mice, swimming was loaded to mice at whose tails each 2% of a weight was attached on the basis of the body weight. The swimming bath was kept at a water temperature of 35 ± 1°C, and 0.1% of a surfactant was added to the water. The swimming time was recorded as a time from the start of swimming until the attachment of the tip of the mouse tail to the bottom. The test solution was orally administered once a day for 3 days, and the forced swimming exercise was loaded one hour after the final oral administration.

In order to examine an effect against swimming fatigue, the mouse to which the forced swimming had been loaded for 10 minutes was removed from the water bath, and orally given the test solution immediately after the removal. The forced swimming test was again performed 15 minutes after the administration to determine the swimming time.

After the test about the above three items, a blood viscosity-lowering effect was examined in order to confirm the effect of far-infrared rays. Significant effects were noted in a stress-relieving test as well as in an anti-fatigue test. It has been demonstrated that the aqueous solution will have excellent effect against broad as well as particular diseases and also various symptoms.

The stress-relieving test was carried out using an aqueous solution containing the graphite silicate.

The graphite silicate has been shown to have a quiet sleep effect through the effect of far-infrared rays and the like. Generally, one feeling stressed is often kept from falling asleep. The graphite silicate is thought to have the possibility of possessing the effect of relieving internal stress because of its quiet sleep effect. On the other hand, GABA is considered to suppress the excessive excitation of cerebral nerves occurring under stress, providing for a feeling of "relaxation". Also, pentobarbital, one of the hypnotics, exerts a hypnotic effect by activating GABA receptors in the brain. Accordingly, it was determined, using the aqueous solution containing the graphite silicate, whether the solution extends sleep duration. If the pentobarbital extends the sleep duration and also has sedative and stress-relieving effects, the aqueous solution will further extend the sleep duration to exert an excellent quiet sleep effect.

A test solution was prepared by adding 10% of graphite silicate powder based on the amount of purified water, and, 1, 3, and 5 days after the addition, the supernatant was used for the test. For the test of determining an effect of the aqueous solution containing the graphite silica on pentobarbital sleep, male mice (8-week old) were used, and allowed to ingest the test solution ad libitum for 3 days; on only the final day, the solution was orally administered (2 mL/kg) one hour before the test. One hour after the administration of the test solution, pentobarbital (50 mg/kg) was intraperitoneally given to determine sleep duration.
Here, the sleep duration was to be a time from the loss of righting reflex until its recovery. As a result, the test solution was evidently noted to have sedative and stress-relieving effects, extending the sleep duration.

Then, a test for examining a blood viscosity-lowering effect of the aqueous solution containing the graphite silicate was carried.

The graphite silicate has been noted to have a blood flow-promoting effect (an effect of making blood smoothly flow) through the effect of far-infrared rays and the like. Accordingly, in order to confirm the promoting effect, the blood viscosity-lowering test was performed using the aqueous solution containing the graphite silicate.

The test solution used here was prepared by adding 10% of graphite silica powder based on the amount of purified water, and, 1, 3, and 5 days after the addition, the supernatant was used for the test.

For studying the blood viscosity-lowering effect, blood was drawn from the ventral aorta of male Wistar rats (330 to 400 g) under ether anesthesia, to which 3 L/ml of a 40 o saline solution of EDTA dipotassium salt was added, as an anticoagulant, per ml of the blood. The resultant blood was subjected to centrifugation (3,000 rpm, 5 min) at 4°C for separation into a supernatant and a red blood cell layer. The supernatant was further subjected to centrifugation (3,000 rpm, 15 min) at 4°C, and the resultant supernatant was used as plasma . The red blood cell layer and the plasma from several rats were mixed to determine the hematocrit of the red blood cell layer. The plasma was added so as to provide a hematocrit of red blood cell layer of 45%, which was then used as a blood for blood viscosity determination.

In order to perform a blood viscosity-lowering test, 50 L of the test solution was added to 1 mL of the blood prepared in 1), followed by incubation at 37°C for 60 minutes. There was fractionated 0.5 mL of the incubated blood to carry out viscosity determination at a shear rate of 7.5 s using a blood viscosity measuring instrument. As a result, when the lowering rate of blood viscosity was calculated as the proportion of blood viscosity after test solution addition to the viscosity without the addition, marked blood viscosity lowering was noted.

The aqueous colloid of graphite silica particles may be therefore used as medicine, or mixed e.g. in drinkable preparations, soft drinks, or health foods.

Further, additives for preparation which may be used in these cases include, for example, a flavor, a coloring agent, a pH regulator, a softening agent, and a flavoring agent, which may be employed in a combination of two kinds or more.

Diseases for which the aqueous colloid of graphite silica particles is intended include, for example, brain and nervous diseases (Alzheimer disease, Parkinson disease, cerebrovascular disease, etc.), lung diseases (pulmonary oxygen intoxication, respiratory distress syndrome, airway disease, etc.), myocardial ischemi-reperfusion injury, myocardialinfarction, vascular diseases (arteriosclerosis, etc.), gut mucosal damages (digestive ulcer, etc.), liver disease, renal diseases (nephritis, renal insufficiency, etc.), eye diseases (cataract, retinal disease, etc.), skin diseases (dermatitis, photosensitivity, etc.), diabetic disease, immune diseases (AIDS, autoimmune disease, etc.), and cancer. This medicine can be also used as an age resister.

In these cases, the dosage and administration frequency of the medicine are not particularly restricted, and can be selected as appropriate depending on various conditions such as a type of disease to be prevented or treated, route of administration, patient's age and body weight, severity of symptom or disease, and the like. In addition, the medicine may be used in a combination with the active ingredient of another medicine.

Further, the aqueous colloid in this Example can be mixed in foods or cosmetics to prevent the deterioration of foods or cosmetics due to radicals e.g. of active oxygen. In the field of cosmetics, the aqueous colloid of graphite silica particles emitting far-infrared rays may be used alone as a bath agent because it can be expected to protect and conserve the skin by suppressing or prevent the occurrence of radicals due to external factors such as ultraviolet radiation. The aqueous colloid may be also used in a combination with a flavor, a moisturizer, and the like.

Pharmacological actions of the aqueous colloid of graphite silicaparticles are further added as described below:
1) Inflammation-suppressing action: The aqueous colloid of graphite silica particles has sufficiently suppressed, in the rat foot edema method using carrageenan as a model of acute inflammationl, the swelling of foot when a foot swelling volume inhibition rate is determined.
2) Carcinogenetic promoter (TPA)-inhibiting action: The aqueous colloid has suppressed, in Hela cells, cellular phospholipid synthesis promotion due to the carcinogenic agent (TPA) with a high inhibition rate. Similarly, the aqueous colloid has inhibited virus (EBV) activation in EBV latent infected human lymphoblast cells.

3) Diabetic complication-suppressing action (associated with diabetic complication treatment): The aqueous colloid has been shown to inhibit the activity of aldose reductase (AR), and can be therefore expected to be used in the prevention or treatment of diabetic complications such as cataract.

4) Antiproliferative effect against tumor cells: The aqueous colloid has been shown to have a DNA synthesis-inhibiting effect in cultured human tumor cells.

5) Inhibitory effect against solid tumor in mice: Mice were inoculated with solid tumor to develop cancer, and allowed to ingest, ad libitum, the aqueous colloid of graphite silica particles for 7 days to examine a change in the wet weight of the solid tumor.

As a result, the determination of a tumor inhibition rate (a reduction rate of tumor weight) in the mice revealed an apparent decrease in tumor. Similarly, an anti-cancer agent (500 mg/kg/day) was administered for 3 days, resulting in a particularly high tumor inhibition rate. These results have confirmed the anti-cancer effect of the aqueous colloid of graphite silica particles.
6) Antioxidant action: Hydrogen peroxide and iron sulfate were added to a rat liver homogenate, which was then incubated, resulting in a significant increase in the mass of lipid peroxide. However, when the aqueous colloid of graphite silica particles was further added to the liver homogenate, the weight of lipid peroxide was shown to be decreased. From these results, the aqueous colloid is thought to have a radical-scavenging (-trapping) effect similar to that of vitamin E.

7) Other actions: To 0 . 5 ml of rat liver microsome (0.5 mml prot./ml) were added 0.2 ml of 3mMADP, 0.2 ml of ferric chloride, and the aqueous colloid of graphite silica particles, followed by incubation at 37°C for 10 minutes, resulting in the confirmation of an excellent effect of the aqueous colloid. In addition, to 0.3 ml of rat small intestine brush border membrane-containing cell fluid were added 0.2 ml of water (ultrapure water in the case of a control group), 0.5 ml of a disaccharide (maltose, saccharose, and lactose) solution, and the aqueous colloid of graphite silica particles, followed by incubation, resulting in the confirmation of an excellent effect of the aqueous colloidon small intestine disaccharidase activity.

Then, characteristics of the aqueous colloid of graphite silica particles are described. The graphite silica particles emitting far-infrared rays and unreacted matter dissolved in a concentration of 1 to 5 wt% in water and filtrated with paper are attached to the skin, resulting in the provision of a feeling of viscosity.

In addition, the aqueous colloid is rich in taste as a drink, and delicious. Further, when the aqueous colloid is sprayed on glass such as spectacles and then swabbed, blur was well removed.

Further, when the colloidal state of the aqueous colloid of graphite silica particles was observed using a laser pointer, Tyndall phenomenon (red laser) was confirmed. The nanocolloid was 1 to 500 nm in size, and the hydrogen atom was 0.1nm.

It has been then shown that the aqueous colloid of graphite silica particles emitting far-infrared rays of the present invention has a foundational ant-caries effect against mutans streptococcus (offending bacteria of caries). It has been also verified that the aqueous colloid has an inhibitory effect against glucosyltransferase (Gtase) involved in the fixing of the bacteria.

Further, the graphite silica particles emitting far-infrared rays and the aqueous colloid of graphite silica particles of the present invention can also be optimally applied in the field of pursuing a "whitening" effect including cosmetics. A key point for whitening is a melanogenesis-suppressing effect, which enables the prevention of blotch.

Attention has been therefore focused on the antioxidant action of the aqueous colloid of graphite silica particles, and the suppressing effect against production of melanin causing post-inflammatory pigmentation or senile pigment macule has been verified using tyrosinase activity as an index.
Attention has been therefore focused on the antioxidant action of the aqueous colloid of graphite silica particles, and the suppressing effect against production of melanin causing post-inflammatory pigmentation or senile pigment macule has been verified using tyrosinase activity as an index. Test Examples and Examples

Table of contents and Figures used for Examples
Example 1: Demonstration of no toxic effects.
Example 2: Demonstration of DNA synthesis without any adverse effect.
Example 3: Demonstration of depressed PGE₂ production in large intestine mucosa.
Example 4: Demonstration of a glucose inhibitory effect by small intestine disaccharidase activity.
Example 5: Demonstration of inhibition of a carcinogenetic promoter; Test 1.
Example 6: Demonstration of inhibition of a carcinogenetic promoter; Test 2.
Example 7: Demonstration of inhibition of a carcinogenetic promoter; Test 3.
Example 8: A preventive effect against carcinogenesis in the large intestine.
Example 9: Demonstration of analgesic and edema-suppressing effects: Test 1.
Example 10: Test for demonstration of an inhibition of liver homogenate.
Example 11: Test for demonstration of an effect against offending bacteria of caries.
Example 12: Test for demonstration of inhibition of tyrosinase activity.

### Example 1 : Demonstration of no toxic effects.

An acute toxicity test of the aqueous colloid of graphite silica particles emitting far-infrared rays of the present invention was first carried out.

### 1. Test substance

The aqueous colloid of graphite silica particles of the present invention was used for this test to allow it to be ingested ad libitum for one month. In addition, distilled water was used as control in an untreated group to allow the water to be ingested ad libitum for one month.

### 2. Animals used and breeding conditions

Four-weekoldSD (Crj : CD) ratswerepurchasedfromCharles River Japan, Inc . , and subjected to quarantine and conditioning for 12 to 13 days, and healthy animals were then used for the test. Animals were bred by housing two animals within each stainless-steel hanger cage in a breeding room having a barrier system set at a temperature of 24±2°C, a humidity of 55±10%, a lighting time of 12 hours (from 7 a.m. to 7 p.m.), and a ventilation frequency of 15 to 20/hour. The feed used was a solid one (CE-2 from Clea Japan, Inc.), and allowed to be ingested ad libitum. Eight rats were used in each group.

### 3. Observation and examination items

### 1) General condition and body weight

During a period of observation, the general condition of each animal and the presence of fat were subjected to two daily checks, morning and afternoon. Body weight was measured twice weekly from the starting date of observation until 2 weeks after the starting date and once weekly from 3 weeks after the starting until the end.

### 2) Blood Biochemical Examination

The blood drawn at the end of examination was subjected to centrifugation at 3, 000 rpm for 10 minutes to provide serum. The serum was measured, by the law of the art, on total protein (modified Lowry method), total bilirubin (alkaline azobilirubin method), GOT (AST) activity (Kaemen method), GPT (ALT) activity (Karmen method),alkaline phosphatase activity (ALP, p-nitrophenylphosphate substrate method), γ-Glutamyltranspeptidase activity (γ-GPT, γ-glutamine-p-nitroanilide substrate method), total cholesterol level (COD-DAOS method), triglyceride level (GPO-DAOS method), phospholipid level (enzymic method), and glucose level (glucose oxidase method).

### 3) Autopsy and organ weight measurement

Rats which had been subjected to blood drawing at the end of observation were dissected to visually observe their organs and tissues. After autopsy, the weights of brain, heart, lung (including bronchus) liver, pancreas, kidney, adrenal, and testis.

### 4) Statistical processing

Values from the experimental results obtained were represented as average ± standard error, and Dunnett method was used for a significant test.

In the above test, no example of death due to the free ingestion of the aqueous colloid of graphite silica particles of the present invention for one month is noted in the rats.

No changes in the weight and external appearance of organs due to the administration of the aqueous colloid of graphite silica particles of the present invention was noted compared to those in the control rats. In the blood biochemical examination, the administration of the aqueous colloid of graphite silica particles of the present invention tended to slightly lower the glucose level, but not induced a significant change in other examination items compared to control.

From these results it has been proved that no toxicological effects of the aqueous colloid of graphite silica particles of the present invention was present under the test conditions.

### Example 2: Tumor proliferation-suppressing effects due to DNA synthesis.

Then, in order to determine proliferation-suppressing effects, against various tumor cells, of the aqueous colloid of graphite silica particles of the present invention, its effects on DNA synthesis were examined in cultured cells using the original solution of the aqueous colloid of the present invention and diluted liquids (obtained by diluting the original solution by 1/10, 1/100, and 1/1000 using ultrapure water).

For control was used the ultrapure water. The cells was cultured in an RPMI-1640 medium containing 10% (v/v) of FCS for 48 hours, followed by determining the uptake of ³H-thymidine using a liquid scintillation counter.

Here, a cultured human tumor cell suspension was prepared by suspending, to an appropriate concentration, HGC (human gastric cancer cell: 2x10⁵ cells/ml), HLC (human lung cancer cell: 1x10⁶ cells/ml) , or U937 (human monocytic leukemia cell: 1x10⁶ cells/ml) in the RPMI-1640 medium containing 10% (v/v) of FCS.

As a result, the aqueous colloid of graphite silica particles of the present invention had a significant DNA synthesis-inhibiting effect on each type of the tumor cells.

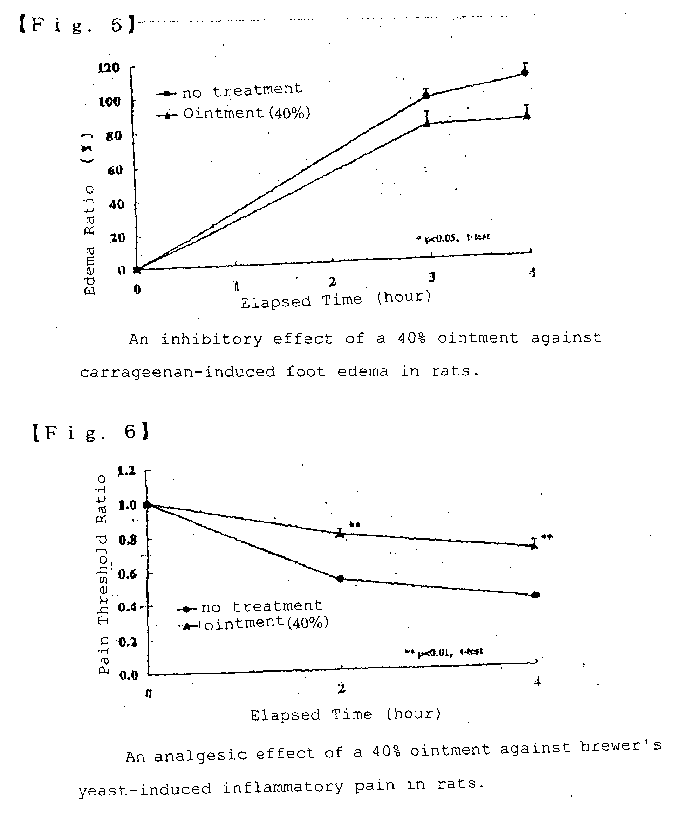

Thus, as the result of studying effects of the aqueous colloid of the present invention on DNA synthesis, the aqueous colloid (of graphite silica particles of the present invention), all of the cultured tumor cells studied were noted to make differences in the DNA synthesis-inhibiting effect depending on differences in the dilute strength thereof. The DNA synthesis-inhibiting effect was shown to be stronger in the cancer cells than in the leukemia cell. (Figure 4).

As described above, this test revealed that the aqueous colloid of the present invention had DNA synthesis-inhibiting effects in the tumor cells. The aqueous nanocolloid of the present invention can be used over a long period of time with almost no side effect and therefore is also thought to have a high use value as an anti-cancer agent.

### Example 3: Demonstration of depressed PGE₂ production in large intestine mucosa.

A test on effects of the aqueous colloid of the present invention on PGE₂ production in large intestine mucosa then attempted. Prostaglandin E₂ produced in the body is considered to be involved in the proceeding of a carcinogenic process, and a substance inhibiting its production has been shown to have a preventive effect against cancer.

Studies in the present invention have confirmed, using a model of large intestine cancer in rats, that the aqueous colloid of the present invention has the effect of suppressing the process of carcinogenesis. Then, in order to elucidate the mechanism of its effect, an effect of the aqueous colloid of the present invention on the production of PGE₂ have been studied in the present invention.

PGE₂ is one of prostaglandins generated from arachidonic acid by cyclooxygenase (COX), and, in carcinogenesis, is considered to be involved e.g. in tumor cell proliferation, apoptosis inhibition, and angiogenesis.

In the present experimental method, 4-week old Wistar male rats (n=6) was subjected to subcutaneous injection of 15 mg/kg of azoxymethane (AOM) 3 times in total, the 1st, 2nd, and 3rd week, and then used in the test at the 8th week. In addition, the aqueous colloid of the present invention was given from 1 week before AOM administration, and allowed to be ingested ad libitum during the test. The concentration of PGE₂ was determined using a kit for measuring PGE₂ (Prostaglandin E₂ Express EIA Kit).

Figure 5 shows the results obtained with regard to the effect of the aqueous colloid of the present invention on PGE₂ production. From this, the PGE₂ production-depressing effect of the aqueous colloid of the present invention has been noted, and it has been proved that depressed PGE₂ production in large intestinemucosa is involved in the suppression of precancerous lesion formation during promotion by the aqueous colloid.

### Example 4: Demonstration of a glucose inhibitory effect due to small intestine disaccharidase activity.

Subsequently, a test was carried out on an effect of the silicon carbide solution on small intestine disaccharidase activity. To 0.3 ml of rat small intestine brush border membrane-containing cell fluid prepared by the law of the art was added 0.2 ml of the solution to be tested (silicon carbide solution: original solution), or an equal amount of ultrapure water in a control group, which was then subjected to preincubation at 37°C for 5 minutes. Then, 0.5 ml of a maltose, saccharose, or lactose solution (final concentration: 42 mM) was added, followed by incubation at 37°C for 20 or 30 minutes for reaction.

Disaccharidase activity was measured quantitatively and in units of mg/min/Mg prot.

The examination of the results made it clear that the addition of each of the three kinds of disaccharides to the rat small intestine brush border membrane-containing cell fluid for incubation at 37°C was noted to produce a significant increase in the disaccharidase activity.

On the other hand, a decrease in the activity of any of the disaccharidases was noted in the rat small intestine brush border membrane-containing cell fluid to which the silicon carbide had been added, leading to the marked suppression of an increase in glucose level. The strength of the suppression of disaccharidase activity in water was in the order of maltase > lactase > saccharase. Thus, the aqueous colloid of the present invention was shown to depress disaccharidase activity.

From these results, it has been suggested that drinking the original solution of the aqueous colloid of the present invention immediately before meal can suppress a marked increase in blood sugar (glucose level) seen after meal in diabetic patients.

A test was also carried out on an effect of the aqueous colloid on insulin secretion after sugar ingestion. A disaccharidase inhibitor is thought to be a useful therapeutic agent for alleviating after-meal hyperglycemia in diabetic patients because the inhibitor suppresses the after-meal hyperglycemia to decrease insulin secretion after meal.

Then, a study was performed on a relationship between the disaccharidase-inhibiting effect of the aqueous colloid and insulin secretion.

In addition, the nanoceramic particle of the present invention and the aqueous colloid of the present invention have been demonstrated to have not any adverse effect on the growth of humans, animals, or plants even when they are used in association with lives of humans or animals, for example, by mixing in a coating for printing and an ink to give antibacterial property, for easing stiff shoulders, by using in a bath agent or various health-enhancing products or accessories, for the raising of a plant or use in a fertilizer therefor, in food, feed, or additive, or in a preservative against mold, or a flavor, a coloring agent, a pH regulator, various softening agents, a flavor, or a dew formation-preventing agent as additive for preparation, which is described successively in Examples below.

### Examples: Experimental Evidence

### Example 5: Demonstration of inhibition of a carcinogenetic promoter; Test 1.

First, the carcinogenetic promoter-inhibitory activity and aldose reductase-inhibitory action (in vitro) of the aqueous colloid of the present invention were studied. The aim of the study is to examine the carcinogenetic promoter inhibitory activity of the inventive aqueous colloid (or its diluted solution) and the aldose reductase inhibitory action associated with a preventive and therapeutic associated with diabetic complications such as diabetic cataract.

The experimental method used the original solution of the aqueous colloid of the present invention and its diluted liquids (1/10andl/100; diluted with ultrapure water) ; however, it used the ultrapure water employed for the dilution in a control group. The carcinogenetic promoter: TPA (12-O-tetradecanoylphorbol-13-acetate) and aldose reductase (derived from human muscle) used products with an extra-pure or higher quality, and the other reagents used had a guaranteed quality.

The result showed that phospholipid synthesis promotion in HeLa cells (MEM liquid medium) by TPA (50 nM) is suppressed by a method taking inhibitory activity against phospholipid synthesis promotion by TPA as an index. Each of the aqueous colloids of the present invention was prepared as an original solution and solutions diluted to 1/10 and 1/100, and the aqueous nanocolloids of the present invention were each added to 0.15 ml of the cells. The cells were cultured for 48 hours by the method of H. Nishino et al. to examine the efficacy. An inhibition rate against uptake of 32pi into phospholipid was represented in %, and determined using a liquid scintillation counter.

Measurements were presented as a mean of duplicate experimental values.

### Example 6: Demonstration of inhibition of a carcinogenetic promoter; Test 2.

Test 2 employed a method using, as an index, an efficacy to suppress the activation of Epstein Barr virus (EBV) by TPA. This method involves adding TPA (32 pM) and 0.15 ml of each of the aqueous nanocolloids of the present invention (the original solution and its liquids diluted by 1/10 and 1/100) to the EBV latent infected human lymphoblast-like cell line Raji cell for incubating 48 hours, then staining EBV-early antigen (EBV-EA) using a indirect fluorescent antibodymethod, calculating an incidence rate (%) of EBV-EA relative to a positive control when the incidence rate of positive cells is defined as 100, and converting the resultant value to an inhibition rate.

### Example 7: Demonstration of inhibition of a carcinogenetic promoter; Test 3.

In Test 3, aldose reductase-inhibiting activity was examined. Thus, 0. 1 ml of a 100 mM dl- glyceraldehyde solution was added to 0.5 ml of each of the aqueous colloids of the present invention (the original solution and its liquids diluted by 1/10 and 1/100) and 0.1 ml of a 3% phosphate buffer solution of aldose reductase, followed by measuring the change of absorbance at 340 nm for 180 seconds to calculate an inhibition rate%. In this respect, measurements were presented as a mean of duplicate experimental values.

As the result of examining a carcinogenetic promote-suppressing effect using an efficacy to inhibit the promotion of cellular phospholipid metabolism by TPA using as an index as described above, the original solution has been shown to have an inhibition rate as high as 69%. However, the aqueous colloid of the present invention diluted to 1/10 and 1/100 have not been to have high inhibitory activities.

Also, as the result of examining the effect using an efficacy to inhibit the activation of Epstein Barr virus (EBV) by TPA using as an index as described above, the original solution has been shown to have a high suppression activity inhibition rate: 70%; however, the aqueous colloid of the present invention diluted by 1/10 has not been to have a high suppression activity.

The above results have shown that the original solution of the aqueous colloid according the present invention has a strong carcinogenetic promoter-suppressing effect while the aqueous colloid according the present invention has not, when diluted, a suppression activity. However, it was clear that the original solution of the aqueous colloid of the present invention strongly inhibits a carcinogenetic promoter.

Further, as the result of examining an aldose reductase-inhibiting activity as described above, the original solution has been shown to have a high AR-inhibiting activity (inhibition rate: 79%). However, the aqueous colloid according the present invention appeared to have, when diluted by 1/10 or 1/100, an insufficient AR-inhibiting activity.

The above results have has demonstrated that the original solution of the aqueous colloid of the present invention may have a significant preventive effect against the later-described diabetic complications such as cataract because of the AR-inhibiting effect thereof.

### Example 8: A preventive effect against carcinogenesis in the large intestine.

Then, an exemplary test about a preventive effect of administration of the aqueous colloid of the present invention against azoxymethane-induced large intestine cancer in rats is described.

The purpose of this test is to examine whether the ingestion of the aqueous colloid of the present invention can reduce carcinogenic risk in a rat large intestine carcinogenesis model by determining the incidence of aberrant crypt foci (ACF) in the large intestine mucosa considered as an initial intermediate index for in the developmental process of large intestine cancer and ornithine decarboxylase activity (ODC) considered as a biochemical index for carcinogenesis promotion.

The experimental method involves subjecting 4-week old Wistar male rats (n=6) to subcutaneous injection of 15 mg/kg of azoxymethane (AOM) 3 times in total, the 1st, 2nd, and 3rd week and assessing the number of premalignant lesions occurring on the large intestine mucosa and the incidence of aberrant crypt foci (by staining the large intestine with a 0.2% methylene blue stain after 8 weeks solution, followed by observation under a stereoscopic microscope (x40)).

Further, ornithine decarboxylase activity in the large intestine mucosa considered as a biochemical index for carcinogenesis promotion was measured according to the method described by Garewal et al. In this respect, the aqueous colloid of the present invention was given from one week before the administration of AOM, and allowed to be ingested ad libitum during a period of test.

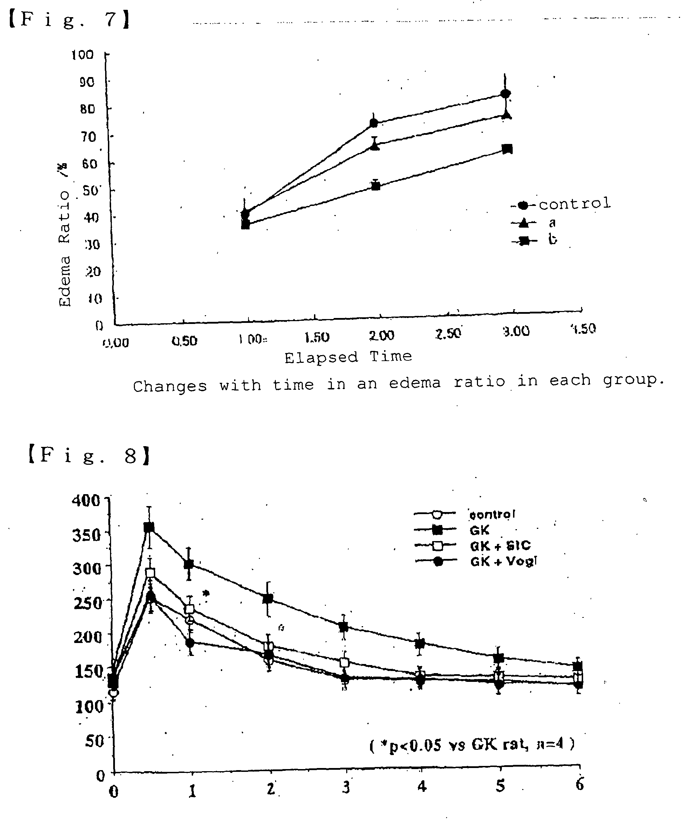

The examination of the result made it clear that the average body weight of rats determined was 263±21 g in the control group, 248±21 g in the AOM group (Figures 6 and 7), and 252±19 g in the group of AOM + the nanocolloid of the present invention, showing no significant change.

Also, as the result of examining the number of ACF indicating precancerous lesions, the number of ACF was 175.5±25.4 in the AOM group and 138.4±22.4 in the group of AOM + the nanocolloid of the present invention, showing a decrease of 22%.

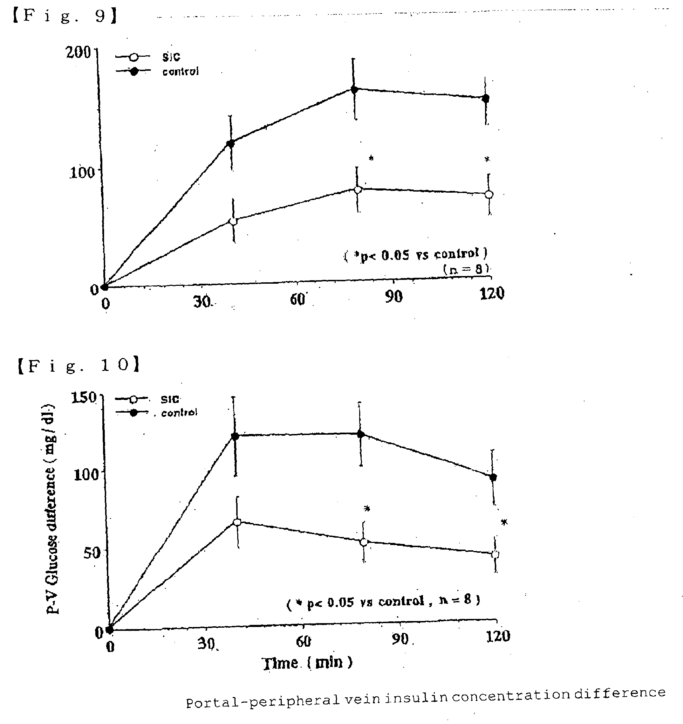

Aberrant crypt foci larger than the average per lesion were also decreased by the administration of the aqueous colloid of the present invention. (See Figure 8)

In addition, the number of large ACF (having sizes equivalent to 4 or more common ACF) was 29.8±7.4 in the AOM group and 19.2±6.4 in the group of AOM + the nanocolloid of the present invention. The ODC activity value (Figure 9) was noted to be significantly decreased in the AOM group compared to that in the group of AOM + the colloid of the present invention.

The above results have showed that the aqueous colloid of the present invention decreases the number of AOM-induced large intestine cancer to be generated. Further, since it decreased the ODC activity value, the aqueous colloid of the present invention is thought to have the possibility of preventing the progression to a less differentiated cancer, which has demonstrated that it exhibits a strong inhibition in the stage of initiation of large intestine carcinogenesis. Example 9: Test for demonstration of analgesic and edema-suppressing effects.

Then, an analgesic effect of the aqueous colloid of the present invention in mice was studied using a writhing reaction induced by acetic acid.

In the observation of the behavior of twisting in response e.g. to a pain due to acetic acid irritation in the peritoneal cavity (writhing), when the administration of an agent suppresses the writhing reaction, the agent can be said to have depressed pseudo affective response, assessing it as having an anti-nociceptive effect.

The method used:
1) ddy strain male mice (7-week old; 27 to 38 g; 6 mice per group);
2) drug solution: aspirin in 0.1 M Tris-Hydrochloride buffer as a solvent (pH 7.8);
3) a 0.7% acetic acid solution: 0.7 ml of acetic acid to which saline was added to provide a total amount of 100 ml; and
4) the aqueous colloid of the present invention: 100 ml of the nanocolloid of the present invention to which 0.9 g of sodium chloride was added to make an isotonicsolution.

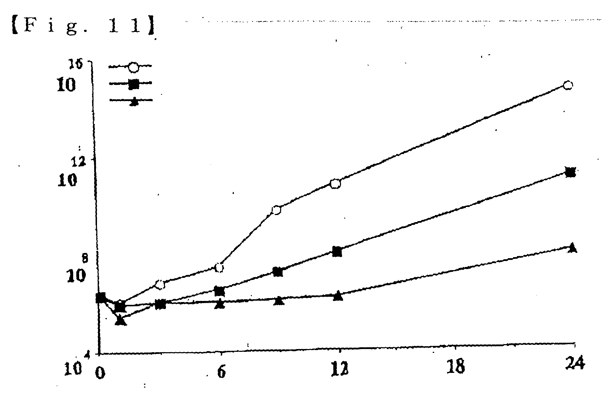

The experimental method involves:
1) dividing mice into 3 groups to subcutaneously administer, to an aspirin (300 mg/kg) treatment group, the aspirin solution, the Tris-Hydrochloride buffer to a control group, and the aqueous nanocolloid (0.1 ml/10 g) to the remaining group;
2) 30 minutes after the administration, intraperitoneally administering the0.7%acetic acidsolution at a dose of 0.1 ml/10 g of body weight; and
3) starting to count the frequency of writhing 10 minutes after the administration of the acetic acid and recording a frequency of writhing during a period of 10 minutes. (See Figure 10)

Figure 10 shows the mean frequency of writhing in each of the groups as the results and or the examination using them. The frequency resulting from the administration of the 0.7% acetic acid solution was 47.1±3.3 (N=6) in the control group, 10.5±1.2 in the aspirin (300 mg/kg) group, and 27.8±5.4 in the group of the aqueous colloid of the present invention which showed a significant decrease in the frequency compared to the control group.

From these results, aspirin and the aqueous nanocolloid has been shown in the acetic acid writhing method to have anti-nociceptive effects, demonstrating that the aqueous colloid according the present invention has an analgesic effect against chemical irritation and an anti-inflammatory effect against intraperitoneal inflammation. In this respect, the above test was carried out using, for each group, six mice, in which similar results were obtained, showing an extremely high reliability of the data.

### Example 16: Test for demonstration of inhibition of liver homogenate.

Then, an antioxidant test was carried out using the aqueous colloid of the present invention in a liver homogenate. To 0.5 ml of a rat liver homogenate prepared by the law of the art was added 0.1 ml of the water to be tested (the aqueous silicon oxide: original solution), or an equal amount of ultrapure water in a control group, followed by preincubation at 37°C for 10 minutes.

Subsequently, 0.2 ml of hydrogen peroxide (final concentration: mM) and 0.2 ml of iron sulfate (final concentration: 0.66 mM) were added, followed by incubation at 37°C for 20 minutes. The amount of the lipid peroxide generated was quantitated, after addition of TBA (2-thiobarbituric acid) reagent, according to the method described by Uchiyama et al. and represented in units of nmol MDA/mg.

The examination of the results confirmed that the addition of hydrogen peroxide and iron sulfate to the liver homogenate followed by incubation at 37°C resulted in a marked increase in the amount of lipid peroxide. However, the further addition of the aqueous silicon carbide resulted in suppressing an increase in the amount of lipid peroxide in the liver homogenate.

The production of lipid peroxide as described in the above test has been reported to be reduced by antioxidants, catalase and glutathione peroxidase (GSH-px), but not by SOD. In addition, the aqueous silicon carbide had no effect of increasing catalase and GSH-px activities, which is not given in the data.

From the above results, it is thought that the aqueous silicon carbide has a radical-scavenging effect similar to that of vitamin E.

### Example 17: Test for demonstration of an effect against offending bacteria of caries.

It has been then shown that the aqueous colloid of graphite silica particles of the present invention has a foundational ant-caries effect against mutans streptococcus (offending bacteriaof caries). It has been also verified that the aqueous colloid has an inhibitory effect against glucosyltransferase (Gtase) involved in the fixing of the bacteria.

Thus, the offending bacteria of caries, first, used Streptococcus mutans MT8148R, and was aerobically cultured employing a brain heart infusion (BHI) agar medium (DIFCO). In order to examine an growth-inhibiting effect against the offending bacteria of caries, a BHI liquid medium was inoculated with Streptococcus mutans MT8148R and aerobically cultured at 37°C for 2.4 hours to make a bacterial suspension; further 50 µl of a sample solution blank: sterilized purified water, sample:SIC 0-fold (standard) or 10-fold (concentration 10 times higher than standard) and 50 µl of the sample solution were added, and aerobically cultured at 37°C.

The incubation was carried out for a prescribed time, and the culture fluid was then diluted, smeared on a BHI medium, and cultured for 48 hours before determining colony forming units (CFU) /ml at each sample concentration and reaction time to examine a Gtase-inhibiting effect, followed by preparing a sample. Adherent, insoluble glucan was separated, and absorbance at 490 nm was then measured using a phenol-sulfuric acid method.

In order to test an antimicrobial activity of the aqueous colloid of graphite silica particles of the present invention against the offending bacteria of caries, the BHI medium containing the aqueous colloid was inoculated with Streptococcus mutans MT8148R, which is then allowed to act for a predetermined amount of time to examine a change with time in vial cell count. As a result, the aqueous colloid 0-fold (standard) containing the graphite silica particles of the present invention had the action to delay, but not inhibit, the proliferation of Streptococcus mutans MT8148R.

In addition, the aqueous colloid 10-fold containing the graphite silica particles of the present invention inhibited the proliferation of Streptococcus mutans MT8148R, but induced little decrease in vital cell count even after 12 hours of action.

These results has shown that the aqueous colloid of graphite silica particles of the present invention has the effect of suppressing the proliferation of the offending bacteria of caries, but the effect is not bactericidal but bacteriostatic. Further, the nanocolloid particle and/or the aqueous colloid of the present invention has been shown to reduce the generation of glucan by Gtase compared to control.

From the above results, it has been suggested that the aqueous colloid of graphite silica particles of the present invention is useful for preventing caries when drunk in a prescribed amount or even when drunk for other purposes because it combined, in the in vitro test, the effect of suppressing the proliferation of the offending bacteria of caries and the activity of inhibiting the production of adherent, insoluble glucan involved significantly in the fixing of the bacteria. Example 18: Test for demonstration of inhibition of tyrosinase activity.

Then, a tyrosinase activity-inhibiting effect useful for "whitening" in the field of cosmetics was examined using the aqueous colloid of graphite silica particles of the present invention.

A key point for whitening is the effect of suppressing melanin production, and the effect enables the prevention of blotch. Thus, making note of the antioxidant effect of the graphite silica particle nanocolloid particle and/or the aqueous colloid of the present invention, its suppressing effect against the production of melanin responsible for post-inflammatory pigmentation or senile pigment macule was tested using tyrosinase activity as an index.

The above aqueous colloid (conc) of the present invention as a basic material, and ascorbic acid and hydroquinone as control agents were used. Tyrosinase used that derived from mushroom (from Sigma Co. , Ltd.: 2,500 U/mg). Specifically, for testing an inhibiting effect against tyrosinase activity, 4 kinds of solutions were prepared.

The solutions were:
(1) a tyrosine solution (0.5 mg/ml); 0.5 ml loading,
(2) a 1/15 M phosphate buffer (pH.6.8) ; 2.0 ml loading,
(3) a tyrosine solution (6.0 mg/100 ml) ; 0.5 ml loading, and
(4) a test solution: the nanocolloid particle and/or the aqueous colloid of the present invention, ascorbic acid, or hydroquinone (0.005, 0.01, 0.05, 0.1, 0.5 mg) dissolved in buffer; 2.0 ml loading.

The solutions of (1) to (4) were mixed to incubate at 37°C for one hour, followed by the measurement of absorbance (At) at 475 nm. In addition, the absorbance obtained by adding the phosphate buffer (2 ml) in place of each test solution to carry out the same operation (Ab) was used as control to determine an inhibition rate. The inhibition rate was calculated from the resultant Ab and At values using the formula: inhibition rate = (Ab - At) / Ab x 100.

As a result, tyrosinase inhibition rates (%) by the loadings of an ascorbic acid and hydroquinone solution were while the inhibition rate by the aqueous colloid of graphite silica particles (conc.) of the present invention was 12.8%.

The tyrosinase inhibition rate of the aqueous colloid of graphite silica particles of the present invention is equivalent to the inhibition rate, in terms of amounts of the control solutions, by a solution containing 0.06 mg of ascorbic acid or 0.005 mg of hydroquinone, representing an apparent effect.

Melanin production uses tyrosine, one of the amino acids, as a starting material. Tyrosinase, which was tested in the present test, are an enzyme catalyzing the two-stage reaction of tyrosine to DOPA, the first stage of melanin production, and DOPA to DOPA quinine. Thus, tyrosinase is most important for melanin production. The present test has shown that ascorbic acid and hydroquinone considered to be effective against pigment macule inhibited the tyrosinase activity.

From the above-described results, it has been suggested that the aqueous colloid of graphite silica particles of the present invention has the effect of inhibiting the production of melanin responsible for post-inflammatory pigmentation or senile pigment macule, and act as a bleaching agent.

The advantage of the present invention is, when described as a whole, that the aqueous colloid of graphite silica particles of the present invention can exert excellent effects in lives of all animals including a human and in raising plants.

More specifically, the aqueous colloid of the present invention may efficiently eliminate radicals such as active oxygen. Since it consists essentially of an inorganic substance, is not used as an energy source in the body, and has the characteristics of easy absorbability and easy excretability, the aqueous colloid is excreted to the outside of the body after circulating in the blood and acting as a radical scavenger.

Therefore, the aqueous colloid of the present invention particularly is highly effective when used e.g. in a medicinal active ingredient, an additive for preparation, a food additive, a health food, a cosmetic, or a bath agent.

Particularly, the aqueous colloid can be highly effective when used as a preventive agent for cancer, brain and nervous diseases (Alzheimer disease, Parkinson disease, cerebrovascular disease, etc.), lung diseases (pulmonary oxygen intoxication, respiratory distress syndrome, airway disease, etc.), myocardial ischemi-reperfusion injury, myocardial infarction, vascular diseases (arteriosclerosis, etc.), gut mucosal damages (digestive ulcer, etc.), liver disease, renal diseases (nephritis, renal insufficiency, etc.), eye diseases (cataract, retinal disease, etc.), skin diseases (dermatitis, photosensitivity, etc.), diabetic disease, immune diseases (AIDS, autoimmune disease, etc.), and aging, in each of which active oxygen is involved.

In addition to such medical treatment, the aqueous colloid can be optimal for use in cosmetics, activation of whole plants, fertilizers, food, feed, accessories such as health rings, pigments, dyes, coating (microbial prevention etc.), stationery products, teaching tools, electronic instruments (electromagnetic wave control), suits (blending), and bath agents, can prevent the toxicity of mercury or lead, and is useful for alleviating effects of a harmful electromagnetic wave, etc. emitted from a personal computer or the like.

In plain words, regular use of the aqueous colloid of graphite silica particles of the present invention can prevent pollinosis, atopy, or obesity as a life-style related disease. Further, it can prevent constipation, have an antirheumatic effect, inhibit edema of the body, and neutralize general toxicants.

Further, the aqueous colloid can prevent various allergies, relieve stress, mitigate grief, nerve strain, and rage, defend against all kinds of viruses, prevent the emergence of fungi or mold, suppress pantophobia, and prevent the so-called dotage. In addition, it has significant antidiarrhetic and bactericidal effects, and can cure sleeplessness, prevent oversensitiveness to cold, and confer protection against the toxicity of mercury or lead.

In particular cases, regular use of the graphite silica particles emitting the far-infrared rays and/or the aqueous colloid of graphite silica particles of the present invention has been reported to have effects such as the curing of cold the next day after catching, during which only sneezes and coughs occurred without fever, the curing of oversensitiveness to cold or pollinosis, the curing of fracture, athlete' s foot, breath shortness, or eye strain in a relatively short period of time, and the whitening of the skin in a relatively short period when used as a cosmetic.

## Claims

**1.** A process for producing an aqueous colloid of graphite silica particles emitting far-infrared rays, the process comprising adding graphite silica (black silica) particles emitting far-infrared rays to water.

**2.** The process for producing an aqueous colloid of graphite silica particles according to claim 1, wherein the graphite silica (black silica) particles are composed of 4 to 6% of carbon, 0.35 to 0.55% of magnesium, 70 to 90% of silica, 1.0 to 2.0% of titanium, 0.4 to 0.6% of iron dioxide, 0.06 to 0.1% of sodium, 5 to 8% of aluminum, 0.8 to 2.0% of potassium, 0.01 to 0.03% of calcium, and 0.25 to 0.4% of water.

**4.** The process for producing an aqueous colloid of graphite silica particles according to claims 1 and 2, wherein the graphite silica (black silica) particles emit far-infrared rays with an emissivity of 90 to 99% at ordinary temperature, and emit so-called growth rays on health rays in a wavelength range of 2 microns to 18 microns.

**5.** The process for producing an aqueous colloid of graphite silica particles according to claims 1 to 4, wherein the graphite silica emitting far-infrared rays has a size of 0.3 micron or less.

**6.** The process for producing an aqueous colloid of graphite silica particles emitting far-infrared rays according to claims 1 to 5, wherein the graphite silica emitting far-infrared rays has a size of 0.3 micron or less, and the aqueous colloid is prepared by dissolving the graphite silica in, for example, normal water, and filtering the solution.

**7.** The process for producing an aqueous colloid of graphite silica emitting far-infrared rays according to claims 1 to 6, wherein the graphite silica emitting far-infrared rays used in the aqueous colloid containing graphite silica particles emitting far-infrared rays is prepare by forming, the material into a spherical shape.

**8.** The process for producing an aqueous colloid of graphite silica emitting far-infrared rays according to claims 1 to 7, wherein the aqueous colloid containing graphite silica particles emitting far-infrared rays is used as an antioxidant.

**9.** The process for producing an aqueous colloid of graphite silica emitting far-infrared rays according to claims 1 to 8, wherein the aqueous colloid containing graphite silica particles emitting far-infrared rays is used in anti-inflammatory agents.

**10.** The process for producing an aqueous colloid of graphite silica emitting far-infrared rays according to claims 1 to 9, wherein the aqueous colloid of graphite silica particles emitting far-infrared rays is used in analgesics.

**11.** The process for producing an aqueous colloid of graphite silica emitting far-infrared rays according to claims 1 to 10, wherein the aqueous colloid containing graphite silica particles emitting far-infrared rays is used in anti-cancer agents.

**12.** The process for producing an aqueous colloid of graphite silica emitting far-infrared rays according to claims 1 to 11, wherein the aqueous colloid containing graphite silica particles emitting far-infrared rays is used in inhibitors for aldose reductase associated with diabetic complications.

**13.** The process for producing an aqueous colloid of graphite silica emitting far-infrared rays according to claims 1 to 12, wherein the aqueous colloid containing graphite silica particlesemittingfar-infraredraysisusedin disaccharidase inhibitors.

**14.** The graphite silica particles emitting far-infrared rays, and the aqueous colloid and the production process therefor according to claims 1 to 13, wherein the aqueous colloid containing graphite silica particles emitting far-infrared rays is used in melanin production inhibitors.

**15.** The graphite silica particles emitting far-infrared rays, and the aqueous colloid and the production process therefor according to claims 1 to 14, wherein the aqueous colloid containing graphite silica particles emitting far-infrared rays is used in anti-allergic agents.

**16.** The graphite silica particles emitting far-infrared rays, and the aqueous colloid and the production process therefor according to claims 1 to 15, wherein the aqueous colloid of graphite silica particles emitting far-infrared rays is used in a mixture with healthy drinks such as drinkable preparations or soft drinks, for example.

**17.** The graphite silica particles emitting far-infrared rays, and the aqueous colloid and the production process therefor according to claims 1 to 16, wherein the aqueous colloid containing graphite silica particles emitting far-infrared rays is used in various human or livestock foods or as an additive for the foods including a flavor or a coloring agent.

**18.** The process for producing an aqueous colloid of graphite silica particles emitting far-infrared rays according to claims 1 to 17, wherein the aqueous colloid of graphite silica particles emitting far-infrared rays is used in, for example, fertilizers for raising various plants and/or improving soil.

**19.** The process for producing an aqueous colloid of graphite silica particles emitting far-infrared rays according to claims 1 to 18, wherein the aqueous colloid containing graphite silica particles emitting far-infrared rays is used in a mixture with various cosmetics such as nourishing creams or cleansing creams, for example, and employed in bath agents or the like.
